# EUROPEAN PATENT APPLICATION

(11) **EP 4 588 565 A1**
(43) Date of publication of application: **23.07.2025**
(21) Application number: 24305105.9
(22) Date of filing: 16.01.2024
(51) Int. Cl.: B01J 35/30, B01J 23/36, B01J 35/61, B01J 37/08, B01J 37/18, C07C 29/153, B01J 37/34, B01J 21/06, C07C 1/12

(54) **ORGANOMETALLIC PREPARATION METHOD OF SUPPORTED RHENIUM CATALYST, SUPPORTED RHENIUM CATALYSTS AND CO2 HYDROGENATION USING SAID CATALYSTS**

(71) Applicant: TOYOTA JIDOSHA KABUSHIKI KAISHA, Toyota-shi, Aichi-ken, 471-8571 (JP); ETH Zurich, 8092 Zürich (CH)
(72) Inventor: NGUYEN, Phuc Hai, 1140 BRUSSELS (BE); COPERET, Christophe, 8092 ZURICH (CH); EHINGER, Christian, 8092 ZURICH (CH); FLORIN, Andri, 8092 ZURICH (CH)
(74) Representative: Cabinet Beau de Loménie

(57) **Abstract**

A method for manufacturing a catalyst for conversion of carbon dioxide via catalysis, comprising a support and catalytic sites provided on the support for the catalysis, the method comprising establishing
- an interaction between a dissolved organometallic compound of Re and the support for obtaining precursors of the catalytic sites on a surface of the support, and
- a conversion of the precursors into the catalytic sites.

## Description

### BACKGROUND OF THE INVENTION

### 1. Field of the Invention

The invention relates to a catalyst for synthesizing methanol from carbon dioxide and hydrogen, a use of and a manufacturing method for the catalyst as well as a flow reactor for conversion of carbon dioxide, in particular for synthesizing methanol from carbon dioxide and hydrogen comprising the catalyst.

### 2. Description of Related Art

The reduction of carbon dioxide using hydrogen is a key process in the production of carbon neutral fuels. The synthesis of methanol from carbon dioxide and hydrogen is such a process.

For a low temperature synthesis of methanol from carbon dioxide and hydrogen Ting et al., ACS Catalysis, Volume 9, Issue 4, Pages 3685 - 3693, 5 April 2019 shows a catalyst with catalytic sites of Re on a support made from TiO₂. Slow kinetics and significant catalyst loss is observed in the known low temperature synthesis even with these catalysts.

For catalysts of this kind Yang et al., ACS Catalysis 2019, 13 (15), 10364-10374 (DOI: 10.1021/acscatal.3c01735) investigates effects of a size of the catalytic sites.

With prior art catalysts at least some of the following problems or drawbacks are observed:
- a low CO₂ conversion and/or low methanol production rate
- a low efficiency of catalytic sites on a support,
- an unreliable methanol production rate,
- a need of an expensive metal element source in significant amounts to provide catalytic sites.

### SUMMARY OF THE INVENTION

It is an object of the invention to improve the synthesis of methanol from carbon dioxide and hydrogen. It is an object of the invention to allow an excellent catalyst performance in the synthesis of methanol from carbon dioxide and hydrogen. It is an object of the invention to efficiently provide a suitable catalyst for this purpose. It is an object of the invention to address aforementioned problems or drawbacks of the prior art.

An aspect of the invention is a method for manufacturing a catalyst for conversion of carbon dioxide via catalysis, in particular a catalyst for synthesizing methanol from carbon dioxide and hydrogen via catalysis, comprising a support and catalytic sites provided on the support for the catalysis, wherein the method comprises establishing
- an interaction between a dissolved organometallic compound of Re and the support for obtaining precursors of the catalytic sites on a surface of the support, and
- a conversion of the precursors into the catalytic sites.

In contrast to prior art concepts, an organometallic chemical reaction is a basis for manufacturing a catalyst according to the invention. The reaction is in the field of surface organometallic chemistry. In other words, the method comprises a conversion of a dissolved organometallic compound of Re, which includes an interaction between the dissolved organometallic compound of Re and the support for obtaining a surface of the support with precursors of the catalytic sites, and obtaining the catalytic sites from a conversion of the precursors into the catalytic sites. Suitable solvents for the dissolving the organometallic compound of Re are commonly known and are commonly used in absence of moisture to have the dissolved organometallic compound of Re. Between 0.1 and 3.5 weight % Re, preferably between 0.1 and 2 weight % Re, particularly preferably between 0.15 and 0.9 weight % Re per total weight of the support may be deposited onto the support. The weight % values are based on the weight of Re atoms and the weight of the support used for the interaction even if Re is present in a bound, in particular an oxide structure on the surface. The catalytic sites may be nanoparticles, e.g. below 1 nm in size, on the support surface in case of a conversion with a reducing gas or may be oxidic sites. Clustering of catalytic sites may or may not be established depending on the concentration of deposited Re. However the invention ensures homogeneously distributed catalytic Re sites. A homogeneous distribution, e.g. in the form of atoms or small clusters or monodispersed nanoparticles, results from the invention's method.

Particular interactions with an organometallic compound of Re and an inorganic oxide material which may serve as a support in the invention are described in e.g. in Salameh et al., Angew. Chem., Int. Ed.2007, 46, 3870-3873; Valla et al., J. Am. Chem. Soc.2016, 138, pp. 6774-6785 or Moses et al., J. Am. Chem. Soc. 2007, 129, 28, pp. 8912-8920. Alkyl-ReO₃ complexes are known and described in Herrmann et al., Acc. Chem. Res. 1997, 30, 4, pp. 169-180 and are specific organometallic compounds of Re. According to an aspect of the invention hydrocarbyl-trioxo-rhenium, in particular alkyl-trioxo-rhenium, is used as the organometallic compound. According to a particular aspect of the invention a monomeric state of the dissolved organometallic compound advantageously suppresses a formation of Re clusters on the support and allows efficiently establishing single atom catalytic sites and related catalytic efficiency. A preferable alkyl-trioxo-rhenium is methyl-trioxo-rhenium. The organometallic compound can be deposited onto the support so that the interaction is established in a vapor deposition on the support before the establishing of the conversion. This means establishing the interaction by exposing a surface of the support to a vapor phase containing the organometallic compound of Re. Then the vapor phase is removed between the establishing of the interaction and the establishing of the conversion. Methyl-trioxo-rhenium is particularly suitable for this vapor deposition concept. According to an alternative preference the organometallic compound, in particular alkyl-trioxo-rhenium, can be handled in a non-protic, preferably an organic solvent and applied to the support in a state of being dissolved in the solvent. The organic solvent is preferably ether- or hydrocarbon-based solvent, most preferably benzene. According to a preference alkyl-trioxo-rhenium dissolved in benzene is used as the organometallic compound. Suitable measures for handling the organometallic compound are commonly known measures in organometallic chemistry for handling an organometallic compound. Such measures include stirring to allow an interaction (in case of the invention particularly the interaction with the support), using the solvent in a dried (demoisturized) state, working under inert gas, in particular dried inert gas like dried nitrogen or dry inert gas like argon, and ensuring a protection against moisture and/or oxygen. Accordingly, in the method according to the invention the interaction may be established in a slurry with a solvent containing the support in a dispersed state and the organometallic compound of Re in a dissolved state and the solvent may be removed between the establishing of the interaction and of the establishing of the conversion. Further preferred measures are at least one of
- protection against moisture in any operation between and any operation of the establishing of the interaction and the establishing the conversion,
- protection against oxygen at least in the establishing of the interaction and up to at least the start of the conversion of the precursors into the catalytic sites or even until and intermediate state of or the end of the conversion of the precursors into the catalytic sites, and
- establishing a reducing atmosphere at least in a final part of the conversion of the precursors into the catalytic sites, wherein it is an option to combine alternating periods, i.e. at least two periods, one in an oxygen containing atmosphere followed by one in a reducing atmosphere as periods of the conversion of the precursors into the catalytic sites.

Titanium dioxide may be used as the support in the invention. The specific surface area (SSA) of the support used for the above mentioned interaction may be between 30 m²/g and 250 m²/g, preferably up to 200 m²/g, more preferably between 40 m²/g and 190 m²/g and particularly preferably at least 50 m²/g.

The support may be any known support for a catalyst (a catalyst for conversion of carbon dioxide via catalysis and/or for synthesizing methanol from carbon dioxide and hydrogen via catalysis, e.g. an inorganic support, preferentially an oxide or sulfide based support, more preferentially an oxide based support. Support examples are alumina, titanium dioxide and perovskites. Titanium dioxide is a preference. With regard to the crystal structure of titanium dioxide anatase is a preference. The support may be conditioned from a suspension, and the suspension may be preferably sonicated, which suppresses agglomeration of particles of the material of the support. It is preferable to condition the material of the support with an aqueous dispersion using ultrasonication and stirring and further drying and/or calcining e.g. between 400°C and 650°C for 0.2 to 3.0 hours, preferably between 450°C and 600°C for 0.5 to 2.75 hours. Conventional techniques may be used for these operations. The calcination may be performed in a flow of synthetic air under exclusion of moisture. A duration longer than 3 hours may result in loss of surface area.

A support which exhibits Lewis acid sites is suitable, like titanium dioxide. The monomeric dissolved organometallic compound and the Lewis acid sites and the solvent allow establishing the above mentioned interaction as an interaction in the absence of moisture. For this purpose the solvent is preferably one which does not interact itself with the support. A basis for a uniquely structured surface of a highly efficient catalyst is provided with this concept.

Solvent removal which preferably comprises evaporating, preferably under vacuum may be performed between said establishing of the interaction and said establishing of the conversion.

The catalytic sites are obtained from the precursors of the catalytic sites with the conversion from the precursors. For this purpose preferably a heat treatment is used, which may be selected from
a) heating in a dry (demoisturized) oxygen containing atmosphere,
b) heating in a dry (demoisturized) oxygen free and/or reducing atmosphere, and
c) heating in a dry (demoisturized) oxygen containing atmosphere followed by heating in a dry oxygen free and/or reducing atmosphere.

The above heat treatment b) is particularly preferred. A temperature of the heat treatment is preferably between 400°C and 600°C, more preferably between 450°C and 550°C, most preferably between 480°C and 520°C, established after a temperature rise at e.g. between 3°C/min to 7°C/min. Possible settings for the heat treatment include:

| kind of heat treatment | temperature | time (hours) | atmosphere |
|---|---|---|---|
| a) heating in a dry oxygen containing atmosphere, | 400°C to 600°C, preferably between 450°C and 550°C, most preferably between 480°C and 520°C | 2 to 5, preferably 2.7 to 3.2 | inert gas with 20 vol % oxygen |
| b) heating in a dry reducing atmosphere, | 400°C to 600°C, preferably between 450°C and 550°C, most preferably between 480°C and 520°C | 0.25 to 1.0, preferably 0.3 to 0.6 | hydrogen |
| c) = a) followed by b) | as indicated above for a) and b), respectively | | |

Unique features can be observed in catalysts of the invention and catalysts obtainable by the method of the invention. According to an aspect bridging oxygen atoms are present which have both, an adjacent Re atom of the catalytic sites and/or the precursors of the catalytic sites and a metal atom of the support in a structure at the catalytic sites and/or the precursors, respectively. According to an aspect of the invention a catalyst for synthesizing methanol from carbon dioxide and hydrogen via catalysis and/or a catalyst for conversion of carbon dioxide via catalysis, comprises
a support and catalytic sites provided on a surface of the support as an oxidic structure with Re for the catalysis, wherein catalytic sites are provided on the support with a dispersity index DI between 1.00 and 1.20, preferably between 1.05 and 1.1. This catalyst may be obtained with the invention's method. Between 0.1 weight % and 3weight % Re per total weight of the support may be deposited on the support in the catalyst. The weight % values are based on the weight of Re atoms and the weight of the support used for the interaction even if Re is present in a bound, in particular an oxide structure on the surface. The dispersity index DI obtainable in a surface observation is the ratio between surface averaged diameters and number averaged diameters in line with IUPAC recommendations published in Stepto, R.F.T. (2010), Dispersity in polymer science (IUPAC Recommendation 2009). Polym. Int., 59: 23-24 (https://doi.org/10.1002/pi.2748). The averaged diameters of this ratio are averaged diameters calculated based on measured particles as described in Bergeret, G.; Gallezot, P. In Handbook of Heterogeneous Catalysis; Ertl, G., Knozinger, H., Schuth, F., Weitkamp, J., Eds.; Wiley-VHC: Weinheim, Germany, 2008; pp 738-765. According to an aspect of the invention a catalyst for synthesizing methanol from carbon dioxide and hydrogen via catalysis and/or for conversion of carbon dioxide via catalysis, comprises
a support and catalytic sites provided on a surface of the support, as an oxidic structure with Re or an Re nanoparticle, for the catalysis. The catalytic sites are provided on the support as catalytic sites attributable to homogeneously distributed Re sites in the form of atoms or small clusters or monodispersed nanoparticles as a result of the invention's method. Thus the catalyst may be obtained with the invention's method. 0.1 and 3 weight % Re per total weight of the support may be deposited on the support in the catalyst. The weight % values are based on the weight of Re atoms and the weight of the support used for the interaction even if Re is present in a bound, in particular an oxide structure on the surface.

Generally a catalyst is obtainable from the method according to the invention and is an aspect of the invention, preferably with a temperature of the heat treatment between 400°C and 600°C and wherein preferably at most 3 weight %, more preferably between 0.1 weight % and 3 weight % Re per total weight of the support is deposited on the support. The weight % values are based on the weight of Re atoms and the weight of the support used for the interaction even if Re is present in a bound, in particular an oxide structure on the surface.

Further aspects of the invention are a flow reactor for conversion of carbon dioxide, in particular synthesizing methanol from carbon dioxide and hydrogen comprising the catalyst according to the invention and a use of the catalyst according to the invention in a, in particular in the flow reactor to catalyze a reaction for conversion of carbon dioxide, in particular synthesizing methanol from carbon dioxide and hydrogen at a temperature between 100 and 300°C and a pressure between 1 and 35 MPa. In particular known reactors may be equipped with a catalyst according to the invention to catalyze a reaction for conversion of carbon dioxide, in particular synthesizing methanol from carbon dioxide and hydrogen in the reactor at suitable reaction conditions.

Particularly suitable specifications for operating the flow reactor with the invention's catalyst are as follows:

| parameter | preferred range | particularly preferred range |
|---|---|---|
| gas mixture flow (mixed from CO₂, H₂ and Ar) | 1 mL/min - 300 mL/min | 6 mL/min - 100 mL/min |
| temperatures | 100°C - 300□ | 200°C to 260°C |
| pressures | 1 MPa - 35 MPa | 2.4 MPa - 2.6 MPa |
| | | e.g. 2.5MPa |

Examplary dispersed material amounts are between 100 mg and 202 mg catalyst, diluted with 4.5 g to 5.5 g SiC.

### BRIEF DESCRIPTION OF THE DRAWING

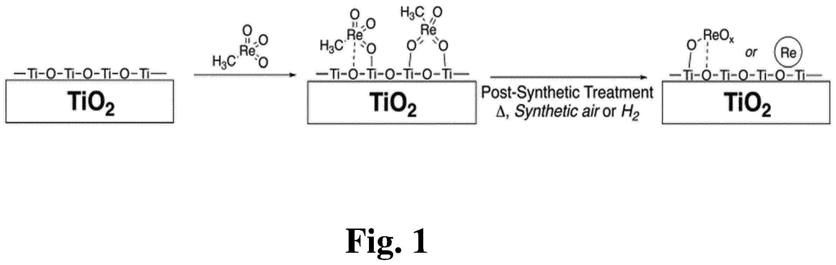

Features, advantages, and technical and industrial significance of exemplary embodiments of the invention will be described below with reference to the accompanying drawing of FIG. 1 which schematically illustrates aspects of a model of structures established in a manufacturing method for a catalyst according to the invention.

### DETAILED DESCRIPTION AND EXAMPLES

FIG. 1 schematically illustrates the basic concept of the invention's method when titanium dioxide is used as a support material and interacted with methyl-trioxo-rhenium dissolved in benzene to from a precursor of a catalytic site followed by a conversion to obtain the catalytic site. The support is schematically illustrated with titanium and oxygen atoms of its surface. The precursor obtained with methyl-trioxo-rhenium on a titanium dioxide support is schematically illustrated. Also as final structures in the illustrated scheme and as catalyst aspects. catalytic sites obtained from alternative conversions (with or without hydrogen) into a catalytic site are schematically illustrated, namely a site with oxidic Re and a site with reduced Re (schematically illustrated as a spherical nanoparticle)..

### Examples

Specific materials used for the preparations indicated below were:
HSA TiO₂ - high specific surface area TiO₂ (CAS: 13463-67-7) with SSA=190 m2/g
P25 - low specific surface area TiO₂ (CAS: 13463-67-7) with SSA=50 m2/g CH₃ReO₃ 99% (CAS: 70197-13-6)

Preparations of samples were performed with the operations described below in atmospheres or vacuum, respectively without exposure to ambient air and ambient moisture. A basis of the sample preparations was a preparation of a support and subjecting the support to an interaction. Specifically a support having a surface with precursors of catalytic sites was prepared by subjecting TiO₂ of each of HSA TiO2 and P25 separately to the following sequence of steps 1 to 5:

| **step** | **details** | **obtained product** |
|---|---|---|
| 1 | calcining 1 g of TiO₂ under a flow of synthetic air (80volume % nitrogen and 20volume % oxygen) | white powder |
| 2 | TiO₂ from step 1 subjected to a vacuum at 10⁻⁵ mbar for 1 h | white powder |
| 3 | in a glovebox filled with Ar: | opaque suspension (purple solid in colourless liquid) |
| | 3a - dissolving CH₃ReO₃ in 5 mL benzene to obtain a colourless solution, | |
| | 3b - dispersing 1 g TiO₂ from step 2 in 10 ml benzene with magnetic stirring (100-200 rpm) to obtain an opaque white suspension, | |
| | 3c - adding the colourless solution to the opaque white suspension and stirring for 5 min at room temperature (100 rpm), | |
| | - then settling for 12 h | |
| 4 | decanting the supernatant colourless liquid and rinsing residual solid two times with 10 mL n-pentane | Wet, purple powder |
| 5 | Drying the powder from step 4 under high vacuum (10⁻⁵ mbar) for 1 h | dry purple powder |

In step 1 HSA was subjected to calcination at 300 °C for 15 min to obtain a specific surface area (SSA) of above 180 m²/g (high surface area in contrast to 50 m²/g which would be obtained with a calcination at 500 °C for 6 hours), or alternatively P25 was subjected to calcination at 500 °C for 6 hours to obtain a specific surface area (SSA) of above 47 m2/g.

### Sample P25-SA and Sample P25-H2

After subjecting P25 to the sequence of steps 1 to 5 for a nominal loading of the support with 1 % Re atom weight (based on the weight of the support) the product obtained from step 5 was subjected to a temperature raise of 5 °C/min up to 500 °C and calcined at 500 °C in a gas flow of 30 mL/min of a dry gas to obtain the respective sample as a white yellowish powder. The dry gas was
- a mix of 80 volume % nitrogen and 20 volume % oxygen in case of sample P25-SA with 3 hours calcination time and
- subsequently hydrogen with 0.5 hours calcination time in case of sample P25-H2.

### Sample HSA-1-SA and Sample HSA-2-H2

After subjecting HSA TiO2 to the sequence of steps 1 to 5 for a nominal loading of the support with 1 % Re atom weight (based on the weight of the support) the product obtained from step 5 was subjected to a temperature raise of 5 °C/min up to 500 °C and calcined at 500 °C in a gas flow of 30 mL/min of a dry gas to obtain the respective sample as a white yellowish powder. The dry gas was
- a mix of 80 volume % nitrogen and 20 volume % oxygen in case of sample HSA-1-SA with 3 hours calcination time and
- subsequently hydrogen for 0.5 hours calcination time in case of sample HSA-2-H2.

### Sample HSA-3-SA/H2

Sample HSA-1-SA was subjected to a temperature raise of 5 °C/min up to 500 °C and calcined at 500 °C for 3h in a gas flow of 30 mL/min of a dry gas which is hydrogen.

### Sample HSA-4-SA

After subjecting HSA TiO2 to the sequence of steps 1 to 5 for a nominal loading of the support with 3 % Re atom weight (based on the weight of the support) the product obtained from step 5 was subjected to a temperature raise of 5 °C/min up to 500 °C and calcined at 500 °C for 3h in a gas flow of 30 mL/min of a dry gas mix of 80 volume % nitrogen and 20 volume % oxygen to obtain sample P25-SA as a white yellowish powder.

### Sample HSA-5-SA

After subjecting HSA TiO2 to the sequence of steps 1 to 5 for a nominal loading of the support with 1 % Re atom weight (based on the weight of the support) the product obtained from step 5 was subjected to a temperature raise of 5 °C/min up to 300 °C and calcined at 300 °C for 3h in a gas flow of 30 mL/min of a dry gas mix of 80 volume % nitrogen and 20 volume % oxygen to obtain sample P25-SA as a white yellowish powder.

### Sample HSA-6-SA

After subjecting HSA TiO2 to the sequence of steps 1 to 5 for a nominal loading of the support with 3 % Re atom weight (based on the weight of the support) the product obtained from step 5 was subjected to a temperature raise of 5 °C/min up to 300 °C and calcined at 300 °C for 3h in a gas flow of 30 mL/min of a dry gas mix of 80 volume % nitrogen and 20 volume % oxygen to obtain sample P25-SA as a white yellowish powder.

### Comparative Sample 'imp.'

A catalyst was prepared according to Yang et al., ACS Catalysis 2019, 13 (15), 10364-10374 (DOI: 10.1021/acscatal.3c01735) and subjected to heating for 3 hours at 500°C under air as a comparative water and moisture based preparation.

### Measurement of actual Re loading

Re loading was determined using ICP-AES (Inductively coupled plasma atomic emission spectroscopy).

### Measurement of conversion rates

CO2 hydrogenation reactions were conducted in a fixed-bed tubular reactor with 9.1 mm inner diameter (PID Eng&Tech). For a catalytic test, 150-350 mg of powdered catalyst was mixed with 4 g of SiC and loaded into the reactor fixed with a by-pass manifold in a glovebox under inert atmosphere. The bypass was flushed with N₂, before introducing the reaction gas mixture H₂:CO₂:N₂ (3:1:1, 50 ml·min⁻¹), heating to 230 °C, and pressurizing to 2.5 MPa. The effluent gas phase was analyzed by GC-FID/TCD (Agilent 7890B), injecting every 30 minutes (1st injection after 30 minutes) using FID for CH3OH and TCD for CO₂, CO, CH₄ and N₂. Flowrates were varied between 6 - 100 ml·min⁻¹ (STP) to probe the effect of different contact times. Every third flowrate restored initial conditions (50 ml·min⁻¹) to track and account for potential deactivation of the catalyst. Each flowrate was sampled 6 times by GC (total of 3 hours). Rates were determined by interpolation to 1% CO₂ conversion using only data points below an inverse gas-hourly-space velocity 1/GHSV < 1.5 e⁻⁸ ml gas/(mol·Re·h) (kinetic regime).

### Results

Data obtained with the above samples, comparative sample and measurements are summarized in the following tables:

| **Sample** | **Support raw material** | **Nominal Re loading w.%** | **Actual Re loading w.%** | **rate mol CO2/ mol Re** / **s** |
|---|---|---|---|---|
| imp. | HSA TiO₂ | 1 | 1.01 | 7.62E-05 |
| P25-SA | P25 | 1 | 0.86 | 2.88E-04 |
| P25-H2 | P25 | 1 | 0.82 | 1.85E-04 |
| HSA-1 SA | HSA TiO₂ | 1 | 0.67 | 2.61E-04 |
| HSA-2 H2 | HSA TiO₂ | 1 | 0.63 | 5.85E-04 |
| HSA-3 SA/H2 | HSA TiO₂ | 1 | 0.75 | 3.90E-04 |
| HSA-4 SA | HSA TiO₂ | 3 | 3 | 6.86E-05 |
| HSA-5 SA (300 °C) | HSA TiO₂ | 1 | 1.1 | 5.25E-05 |
| HSA-6 SA (300 °C) | HSA TiO₂ | 3 | 3 | 1.15E-04 |

| **Sample** | **rate mol CO2/ mol Re / s** | **CO selectivity (%)** | **MeOH selectivity (%)** | **CH4 selectivity (%)** |
|---|---|---|---|---|
| imp. | 7.62E-05 | 69% | 27% | 4% |
| P25-SA | 2.88E-04 | 1% | 2% | 96% |
| P25-H2 | 1.85E-04 | 51% | 29% | 21% |
| HSA-1 SA | 2.61E-04 | 90% | 6% | 5% |
| HSA-2 H2 | 5.85E-04 | 73% | 24% | 3% |
| HSA-3 SA/H2 | 3.90E-04 | 71% | 24% | 4% |
| HSA-4 SA | 6.86E-05 | 9% | 8% | 83% |
| HSA-5 SA (300 °C) | 5.25E-05 | 67% | 20% | 13% |
| HSA-6 SA (300 °C) | 1.15E-04 | 13% | 12% | 75% |

For the above described dispersity index parameter Dispersity index values prior to the conversion step were observed as follows:
- DI=1.03 in the case of HSA-2: 1.10±0.14 nm (N=225);
- DI=1.06 in the case of P25-H2: 1.16±0.19 nm(N=775).

### Discussion

In view of the above it can be concluded that according to an aspect the invention profits from a new concept of depositing Re to provide catalytic sites on a catalyst support. Most performant catalysts are obtained with proper heating temperatures for the conversion of precursors of catalytic sites into the catalytic sites with a properly adjusted Re loading amounts on a support surface.

Although the present disclosure refers to specific exemplary embodiments, modifications may be provided to these examples without departing from the general scope of the invention as defined by the claims. In particular, individual characteristics of the different illustrated/mentioned embodiments may be combined in additional embodiments. Therefore, the description and the drawing should be considered in an illustrative rather than in a restrictive sense.

## Claims

1. A method for manufacturing a catalyst for conversion of carbon dioxide via catalysis, comprising a support and catalytic sites provided on the support for the catalysis,
**characterized in that**
the method comprises establishing
- an interaction between a dissolved organometallic compound of Re and the support for obtaining precursors of the catalytic sites on a surface of the support, and
- a conversion of the precursors into the catalytic sites.

2. The method according to claim 1, wherein the interaction is established in a slurry with a solvent containing the support in a dispersed state and the organometallic compound of Re in a dissolved state and the solvent is removed between the establishing of the interaction and the establishing of the conversion.

3. The method according to claim 1, wherein the interaction is established by exposing a surface of the support to a vapor phase containing the organometallic compound of Re the vapor phase is removed between the establishing of the interaction and the establishing of the conversion.

4. The method according to claim 1, 2 or 3, wherein protection against moisture is provided in any operation between and any operation of the establishing of the interaction and of the establishing of the conversion.

5. The method according to claim 4, wherein protection against oxygen is provided at least in the establishing of the interaction.

6. The method according to claim 5, wherein at least a final part of the conversion of the precursors into the catalytic sites proceeds in a reducing atmosphere.

7. The method according to any one of claims 1 to 6, wherein the organometallic compound of Re is hydrocarbyl-trioxo-rhenium.

8. The method according to any one of claims 1 or 7, wherein the support has a specific surface area between 30 m²/g and 200 m²/g.

9. The method according to any one of claims 1 to 8, wherein bridging oxygen atoms are present which have both, an adjacent Re atom of the catalytic sites and/or the precursors of the catalytic sites and a metal atom of the support in a structure at the catalytic sites and/or the precursors, respectively.

10. The method according to any one of claims 1 to 9, comprising a heat treatment for the conversion of the precursors into the catalytic sites with a temperature between 400°C and 600°C.

11. A catalyst for conversion of carbon dioxide via catalysis, comprising
a support,
catalytic sites provided on a surface of the support as an oxidic structure with Re for the catalysis,
**characterized in that**
among catalytic sites provided on the support with a dispersity index DI between 1.00 and 1.20.

12. A catalyst for conversion of carbon dioxide via catalysis, wherein the catalyst is one obtained from the method according to claim 10 and in which 0.1 and 3 % Re atom weight per total weight of the support is deposited on the support.

13. The catalyst of claim 11 obtained from the method according to claim 10, wherein 0.1 and 3 % Re atom weight per total weight of the support is deposited on the support.

14. A flow reactor for conversion of carbon dioxide comprising the catalyst according to any one of claims 11 to 13.

15. A use of the catalyst according to any one of claims 11 to 13 in a flow reactor to catalyze a reaction for conversion of carbon dioxide at a temperature between 100 and 300°C and a pressure between 1 and 35 MPa.

## Amended claims

### Amended claims in accordance with Rule 137(2) EPC.

1. A method for manufacturing a catalyst for conversion of carbon dioxide via catalysis, comprising a support being titanium dioxide and catalytic sites of Re provided on the support for the catalysis in an amount between 0.1 and 3% Re atom weight deposited on the support per total weight of the support,
wherein the method comprises establishing
- an interaction between a dissolved organometallic compound of Re being alkyl-trioxo-rhenium and the support for obtaining precursors of the catalytic sites on a surface of the support,
- a conversion of the precursors into the catalytic sites, and
- a heat treatment for the conversion of the precursors into the catalytic sites with a temperature between 400°C and 600°C.

2. The method according to claim 1, wherein the interaction is established in a slurry with a solvent containing the support in a dispersed state and the organometallic compound of Re in a dissolved state and the solvent is removed between the establishing of the interaction and the establishing of the conversion.

3. The method according to claim 1, wherein the interaction is established by exposing a surface of the support to a vapor phase containing the organometallic compound of Re the vapor phase is removed between the establishing of the interaction and the establishing of the conversion.

4. The method according to claim 1, 2 or 3, wherein protection against moisture is provided in any operation between and any operation of the establishing of the interaction and of the establishing of the conversion.

5. The method according to claim 4, wherein protection against oxygen is provided at least in the establishing of the interaction.

6. The method according to claim 5, wherein at least a final part of the conversion of the precursors into the catalytic sites proceeds in a reducing atmosphere.

7. The method according to any one of claims 1 to 6, wherein the organometallic compound of Re is hydrocarbyl-trioxo-rhenium.

8. The method according to any one of claims 1 to 7, wherein bridging oxygen atoms are present which have both, an adjacent Re atom of the catalytic sites and/or the precursors of the catalytic sites and a metal atom of the support in a structure at the catalytic sites and/or the precursors, respectively.

9. A catalyst for conversion of carbon dioxide via catalysis, wherein the catalyst is one obtained from the method according to any one of claims 1 to 8 and in which 0.1 and 3 % Re atom weight per total weight of the support is deposited on the support.

10. A flow reactor for conversion of carbon dioxide comprising the catalyst according to claim 9.

11. A use of the catalyst according to claim 9 in a flow reactor to catalyze a reaction for conversion of carbon dioxide at a temperature between 100 and 300°C and a pressure between 1 and 35 MPa.
